(19) Europäisches Patentamt
European Patent Office
Office européen des brevets

(11) **EP 4 228 503 B1**

(12) # EUROPEAN PATENT SPECIFICATION

(45) Date of publication and mention
of the grant of the patent:
**14.05.2025 Bulletin 2025/20**

(21) Application number: **21801204.5**

(22) Date of filing: **18.10.2021**

(51) International Patent Classification (IPC):
**A61B 5/024** *(2006.01)*    **A61B 5/0245** *(2006.01)*

(52) Cooperative Patent Classification (CPC):
**A61B 5/024; A61B 5/02438; A61B 5/0245**

(86) International application number:
**PCT/GB2021/052687**

(87) International publication number:
**WO 2022/084661 (28.04.2022 Gazette 2022/17)**

(54) **METHOD AND SYSTEM FOR DETERMINING A RECOVERY SCORE FOR A SUBJECT**

VERFAHREN UND SYSTEM ZUR BESTIMMUNG EINER WIEDERHERSTELLUNGSBEWERTUNG FÜR EINE PERSON

PROCÉDÉ ET SYSTÈME POUR DÉTERMINER UN SCORE DE RÉCUPÉRATION POUR UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.10.2020 GB 202016555**

(43) Date of publication of application:
**23.08.2023 Bulletin 2023/34**

(73) Proprietor: **Prevayl Innovations Limited**
**Wilmslow SK9 1LD (GB)**

(72) Inventor: **ROBERTAUD, David**
**Manchester Greater Manchester M2 3AZ (GB)**

(74) Representative: **Bird, Samuel Joseph**
**Hanna & Bird IP**
**Suite 4 Stanley House**
**19-23 Crofts Bank Road**
**Urmston M41 0TZ (GB)**

(56) References cited:
**US-A1- 2015 182 129    US-A1- 2016 066 840**
**US-A1- 2016 143 579**

## Description

The present invention is directed towards a method and system for determining a recovery score for a subject. In particular, the present invention is directed towards methods and systems for determining a recovery score using heartrate values of the subject obtained when the user is resting (e.g. sitting or lying down) and standing.

## Background

Wearable articles, such as garments, incorporating sensors are wearable electronics used to measure and collect information from a wearer. Such wearable articles are commonly referred to as 'smart clothing'. It is advantageous to measure biosignals of the wearer during exercise, or other scenarios.

It is known to provide a garment, or other wearable article, to which an electronic device (i.e. an electronics module, and/or related components) is attached in a prominent position, such as on the chest or between the shoulder blades. Advantageously, the electronic device is a detachable device. The electronic device is configured to process the incoming signals, and the output from the processing is stored and/or displayed to a user in a suitable way
A sensor senses a biosignal such as electrocardiogram (ECG) signals and the biosignals are coupled to the electronic device, via an interface. The sensors may be coupled to the interface by means of conductors which are connected to terminals provided on the interface to enable coupling of the signals from the sensor to the interface.

Electronics modules for wearable articles such as garments are known to communicate with user electronic devices over wireless communication protocols such as Bluetooth ® and Bluetooth ® Low Energy. These electronics modules are typically removably attached to the wearable article, interface with internal electronics of the wearable article, and comprise a Bluetooth ® antenna for communicating with the user electronic device.

The electronic device includes drive and sensing electronics comprising components and associated circuitry, to provide the required functionality. The drive and sensing electronics include a power source to power the electronic device and the associated components of the drive and sensing circuitry.

ECG sensing is used to provide a plethora of information about a person's heart. It is one of the simplest and oldest techniques used to perform cardiac investigations. In its most basic form, it provides an insight into the electrical activity generated within heart muscles that changes over time. By detecting and amplifying these differential biopotential signals, a lot of information can be gathered quickly, including the heart rate. Among professional medical staff, individual signals have names such as "the QRS complex," which is the largest part of an ECG signal and is a collection of Q, R, and S signals, including the P and T waves.

Whilst lay persons may not be aware of the clinical aspects and significance of an ECG signal trace, lay persons would usually recognise the general form of such a signal trace, if only as a measure of heart rate.

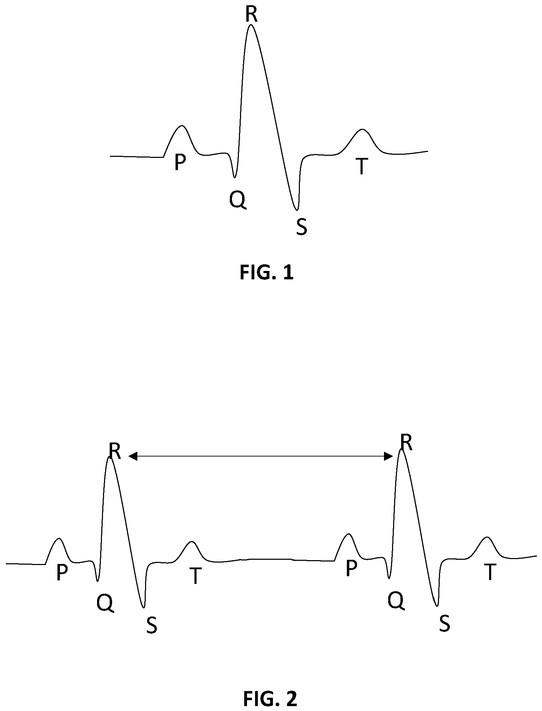

Typically, the detected ECG signals can be displayed as a trace to a user for information. The user may be a clinician who is looking to assess cardiac health or may be a lay user using the electronics module as a fitness or health and wellness assessment device. A typical ECG waveform or trace is illustrated in Figure 1 showing the QRS complex. Figure 2 shows an ECG waveform of two successive heartbeats. The time difference between the two R peaks in the ECG waveform is the inter-beat interval (IBI) also known as the R-R interval. This time is usually expressed in milliseconds. IBI values represent the time between successive heartbeats.

The orthostatic heart rate (OHR) test (and other similar tests) is an established and widely used test for monitoring the fitness level of a subject. OHR test results can indicate whether the subject is stressed, overtired, overtrained, or is ill. OHR tests are widely used in the managing of training of athletes and other individuals.

An OHR test measures the difference between the resting heart rate of the subject and the orthostatic heart rate. The resting heart rate of the subject refers to the heart rate when the user is at rest such as when sitting or lying down in a relaxed position. Generally, the subject is lying down in the supine position. The orthostatic heart rate is the heart rate of the user when standing.

The difference between the resting heartrate and the orthostatic heart rate is normally around 15 BPM for a healthy individual. Generally, however, it is difficult for a user to determine useful information from the OHR test by itself. For example, on separate days a user may record OHR test values of 7, 10, 15 and 20. It may be difficult for the user to determine the significance of these differences in scores. For example, the user may struggle to determine whether an OHR test value of 10 is worse than a test value of 20, and the significance of having an OHR test value of 7 compared to an OHR test value of 10.

United States Patent Application Publication No. US 2016/0066840 A1 discloses a system, device and method of determining a probability of dehydration of a person. The method comprises receiving data of a heart rate of the person; receiving data of the posture of the person; determining that a first posture of the person satisfied first posture criteria for a first predetermined time period; determining that the posture of the person satisfies a similarity threshold with a posture envelope; determining a first heart rate for the person while in the first posture; determining a second heart rate for the person while in the second posture; determining a change in heart rate as a difference between the second heart rate and the first heart rate; determining a first probability of dehydration based at least in part on the change in heart rate; and output the first probability of dehydration.

United States Patent Application Publication No. US 2015/0182129 A1 discloses a method for measuring stress based on the heart rate (HR) and heart rate variability (HRV), and the activity level of the user at various times during the day.

United States Patent Application Publication No. US 2016/0143579 A1 discloses wearable electronic devices, systems and methods for sports performance monitoring.

An object of the present invention is to provide an improved method and system for determining a recovery state of a subject based on the measured heartrate values of the subject.

Summary

According to the present disclosure there is provided a computer-implemented method and system as set forth in the appended claims. Other features of the invention will be apparent from the dependent claims, and the description which follows.

According to a first aspect of the disclosure, there is provided a computer-implemented method of calculating a recovery score for a subject. The method comprises obtaining a pair of heartrate values, a first of the values, $HR_{rest}$, representing the heartrate of the subject in a resting position and a second of the values, $HR_{stand}$, representing the heartrate of the subject in a standing position. The method comprises inputting the pair of heartrate values into a probability density function $f(HR_{rest}, HR_{stand})$ which uses the difference between ($HR_{stand} - HR_{rest}$) and a non-zero constant, $\mu$, that represents a desired value of the difference between the standing and resting heart rates, to generate a recovery score for the subject. In other words, the function determines ($HR_{stand} - HR_{rest}$) - $\mu$. The method further comprises outputting the generated recovery score.

Without being bound to any particular theory, values of $HR_{stand} - HR_{rest}$ have been found to follow a generally normal distribution around an optimum value $\mu$. The present disclosure takes advantage of this by inputting $HR_{stand}$ and $HR_{rest}$ into a probability density function so as to generate a recovery score for the subject. Thus, rather than simply outputting the value of the difference between $HR_{stand}$ and $HR_{rest}$, the present disclosure uses $HR_{stand}$ and $HR_{rest}$ to generate a recovery score that provides an intuitive, unambiguous, indication of the recovery state of the subject.

Here, and throughout this description the "user" and the "subject" may refer to the same or different people. The user may be, for example, a medical professional, fitness instruction or coach (amongst other examples) who is monitoring the performance of the subject. Equally, the subject may be observing their own recovery score.

The function $f(HR_{rest}, HR_{stand})$ divides ($HR_{stand} - HR_{rest}$) - $\mu$ by a non-zero constant $\sigma$ that defines the width of the curve of the distribution generated by the probability density function.

$\mu$ may be proportional to $\sigma$. Or equally, $\sigma$ may be proportional to $\mu$. In some examples, $\mu = 3\sigma$. That is, the optimum recovery score may be equal to three standard deviations ($\sigma$). Without being bound to any particular theory, it has been found that the optimum difference between $HR_{stand} - HR_{rest}$ (i.e. $\mu$) is three standard deviations from the lowest expected difference between $HR_{stand} - HR_{rest}$ and three standard deviations from the maximum expected difference between $HR_{stand} - HR_{rest}$. Thus, setting $\mu = 3\sigma$ is beneficial in increasing the accuracy of the generated recovery score.

$\sigma$ may be determined according to the maximum expected difference between standing and resting heart rates for the subject, $OHR_{max}$. This maximum expected difference is not necessarily the same as the measured difference between $HR_{stand} - HR_{rest}$. $\sigma$ may be determined according to the minimum expected difference between standing and resting heart rates for the subject, $OHR_{min}$. This minimum expected difference is not necessarily the same as the measured difference between $HR_{stand} - HR_{rest}$.

$\sigma$ may be determined by calculating $OHR_{max} - OHR_{min}$.

$\sigma$ may be determined by dividing $OHR_{max} - OHR_{min}$ by a non-zero constant C. C may be equal to the number of standard deviations required to get from $OHR_{min}$ to $\mu$ and from $\mu$ to $OHR_{max}$. In preferred examples, C = 6. This follows from the finding that the optimum difference between $HR_{stand} - HR_{rest}$ (i.e. $\mu$) is three standard deviations from the lowest expected difference between $HR_{stand} - HR_{rest}$ and three standard deviations from the maximum expected difference between $HR_{stand} - HR_{rest}$.

In some examples, $\sigma = (OHR_{max} - OHR_{min})/C$.

$OHR_{max} - OHR_{min}$ may be between 20 and 50, may be between 25 and 40, and may be equal to 40. In some examples, $OHR_{max} = 30$ and $OHR_{min} = 0$.

The values of $\mu$, $\sigma$, $OHR_{max}$, $OHR_{min}$ may be subject specific. One or more of $\mu$, $\sigma$, $OHR_{max}$, $OHR_{min}$ may be determined based on one or more characteristics of the subject. Characteristics include the subject's age, weight, gender, ethnicity, fitness level, diet, medical history, or lifestyle (e.g. whether they are a smoker). The values of $\mu$, $\sigma$, $OHR_{max}$, $OHR_{min}$ may be updated over time as characteristics of the subject change.

$\mu$ may be between 5 and 25. $\mu$ may be between 10 and 20. In some examples, $\mu$ is 15.

The function $f(HR_{rest}, HR_{stand})$ involves determining $S \times \frac{A}{\sigma} \times B^{\left(\frac{(HR_{stand}-HR_{rest})-\mu}{\sigma}\right)^2}$. The function $f(HR_{rest}, HR_{stand})$ is of the form:

$$f(HR_{rest}, HR_{stand}) = S \times \frac{A}{\sigma} \times B^{\left(\frac{(HR_{stand}-HR_{rest})-\mu}{\sigma}\right)^2}$$

That is, $f(HR_{rest}, HR_{stand})$ is equal to S times $\frac{A}{\sigma}$ times ($B$ to the power of $\left(\frac{(HR_{stand}-HR_{rest})-\mu}{\sigma}\right)^2$).

A and B are non-zero constants.

$\mu$ is the non-zero constant that represents a desired value of the difference between the standing and resting heart rates, and acts as a location parameter that translates the peak of the curve of the distribution generated by the probability density function to a location representing an optimum recovery score for the subject.

$\sigma$ is the non-zero scaling constant that defines the width of the curve of the distribution generated by the probability density function.

S is a non-zero constant that acts as a scaling factor.

In some examples, $B = e^{-\frac{1}{2}}$. Here, e is the Euler number, a mathematical constant, approximately equal to 2.72.

The constant S may scale the probability density function such the optimum values of $HR_{stand}$ and $HR_{rest}$ result in a desired maximum recovery score. In some examples, S = $P \times \sigma/A$, wherein P is a non-zero constant that sets the upper limit of the recovery score. In other words, the function may be of the form:

$$f(HR_{rest}, HR_{stand}) = P \times B^{\left(\frac{(HR_{stand}-HR_{rest})-\mu}{\sigma}\right)^2}.$$

In some examples, $A = 1/\sqrt{2\pi}$ and $S = P \times \sigma \times \sqrt{2\pi}$.

Advantageously, the recovery score is unambiguous as it has defined upper and lower limits (e.g. a score of 10 indicates the optimum situation and a score of 0 indicates the worst case). The outputted recovery score can enable the user to determine, for example, whether the subject should continue to train as normal, reduce their training intensity, take a rest day or even seek medical advice.

In some examples, P = 10. More generally, P is not limited to any particular value. P may be, for example, 5, 20, 50, or 100.

In some examples, the function $f(HR_{rest}, HR_{stand})$ is of the form:

$$f(HR_{rest}, HR_{stand}) = S \times \frac{A}{\sigma} \times B^{\left(\frac{(HR_{stand}-HR_{rest})-3\sigma}{\sigma}\right)^2}$$

In some examples, the function $f(HR_{rest}, HR_{stand})$ is of the form:

$$f(HR_{rest}, HR_{stand}) = P \times B^{\left(\frac{(HR_{stand}-HR_{rest})-3\sigma}{\sigma}\right)^2}$$

In some examples, the function $f(HR_{rest}, HR_{stand})$ is of the form:

$$f(HR_{rest}, HR_{stand}) = P \times e^{-\frac{1}{2}\left(\frac{(HR_{stand}-HR_{rest})-3\sigma}{\sigma}\right)^2}$$

In some examples, the function $f(HR_{rest}, HR_{stand})$ is of the form:

$$f(HR_{rest}, HR_{stand}) = 10 \times e^{-\frac{1}{2}\left(\frac{(HR_{stand}-HR_{rest})-15}{5}\right)^2}$$

Obtaining the pair of heartrate values may comprise:

obtaining a first sequence of heartbeat data samples of the subject in a resting position and a second sequence of heartbeat data samples of the subject in a standing position, wherein the heartbeat data samples comprises inter-beat interval, IBI, values representing the time between successive heartbeats;

calculating $HR_{rest}$ from the first sequence of heartbeat data samples; and

calculating $HR_{stand}$ from the second sequence of heartbeat data samples.

Calculating $HR_{rest}$ may comprises dividing 60000 by the average IBI value in milliseconds for the first sequence of heartbeat data samples.

Calculating $HR_{stand}$ may comprise dividing 60000 by the average IBI value in milliseconds for the second sequence of heartbeat data samples.

The method may be performed by a controller for a user electronic device. The user electronics device may further include an interface, coupled to the controller, and arranged to receive signals from an electronics module for a wearable article. The signals may comprise biosignals representing the electrical activity of the heart of the subject. The biosignals may comprise heartbeat data samples of the subject.

The method may be performed by an electronics module for a wearable article. The electronics module may have an output unit for outputting the recovery score. The output may be in the form of an audible, visual and/or haptic feedback. The electronics module may have a display for displaying the recovery score. The electronics module may be a component of a smartwatch for example.

According to a second aspect of the disclosure, there is provided a computer-readable medium having instructions recorded thereon which, when executed by a processor, cause the processor to perform the method of the first aspect of the disclosure.

According to a third aspect of the disclosure, there is provided a system for calculating a recovery score for a subject, the system comprising a processor and a memory, the memory storing instructions which when executed by the processor cause the processor to perform operations comprising:

obtaining a pair of heartrate values, a first of the values, $HR_{rest}$, representing the heartrate of the user in a resting position and a second of the values, $HR_{stand}$, representing the heartrate of the user in a standing position;

inputting the pair of heartrate values into a probability density function $f(HR_{rest}, HR_{stand})$ which uses the difference between ($HR_{stand} - HR_{rest}$) and a non-zero constant, $\mu$, that represents a desired value of the difference between the standing and resting heart rates, to generate a recovery score for the subject. In other words, the function determines ($HR_{stand} - HR_{rest}$) - $\mu$; and

outputting the generated recovery score.

The function $f(HR_{rest}, HR_{stand})$ divides ($HR_{stand} - HR_{rest}$) - $\mu$ by a non-zero constant $\sigma$ that defines the width of the curve of the distribution generated by the probability density function.

**The function** $f(HR_{rest}, HR_{stand})$ is of the form:

$$f(HR_{rest}, HR_{stand}) = S \times \frac{A}{\sigma} \times B^{\left(\frac{(HR_{stand} - HR_{rest}) - \mu}{\sigma}\right)^2}$$

A and B are non-zero constants.

$\mu$ is the non-zero constant that represents a desired value of the difference between the standing and resting heart rates, and acts as a location parameter that translates the peak of the curve of the distribution generated by the probability density function to a location representing an optimum recovery score for the subject.

$\sigma$ is the non-zero scaling constant that defines the width of the curve of the distribution generated by the probability density function.

S is a non-zero scaling constant.

The system may further comprise a display. The display may be arranged to display the generated recovery score.

The system may comprise a user electronics device. The user electronics device may comprise the processor and the memory. The user electronics device may comprise the display and/or other form of output unit for outputting the generated recovery score. The user electronics device may comprise an interface, coupled to the controller, the controller being arranged to receive signals from an electronics module for a wearable article. The controller may be configured to obtain biosignal data for a wearer of the wearable article from the electronics module.

The system may comprise the electronics module for the wearable article. The electronics module may provide biosignal data to a user electronics device comprising the processor and the memory. The electronics module may comprise the processor and the memory. The electronics module may have an output unit for outputting the recovery score. The output may be in the form of an audible, visual and/or haptic feedback. The electronics module may have a display for displaying the recovery score. The electronics module may be a component of a smartwatch.

There is also disclosed an apparatus for calculating a recovery score for a subject, the apparatus comprising a processor and a memory, the memory storing instructions which when executed by the processor cause the processor to perform operations comprising:

obtaining a pair of heartrate values, a first of the values, $HR_{rest}$, representing the heartrate of the user in a resting position and a second of the values, $HR_{stand}$, representing the heartrate of the user in a standing position;

inputting the pair of heartrate values into a probability density function $f(HR_{rest}, HR_{stand})$ which uses the difference between $(HR_{stand} - HR_{rest})$ and a non-zero constant, $\mu$, that represents a desired value of the difference between the standing and resting heart rates, to generate a recovery score for the subject. In other words, the function determines $(HR_{stand} - HR_{rest}) - \mu;$ and

outputting the generated recovery score.

In some examples, the function $f(HR_{rest}, HR_{stand})$ divides $(HR_{stand} - HR_{rest}) - \mu$ by a non-zero constant $\sigma$ that defines the width of the curve of the distribution generated by the probability density function.

The function $f(HR_{rest}, HR_{stand})$ may be of the form:

$$f(HR_{rest}, HR_{stand}) = S \times \frac{A}{\sigma} \times B^{\left(\frac{(HR_{stand} - HR_{rest}) - \mu}{\sigma}\right)^2}$$

A and B are non-zero constants.

$\mu$ is the non-zero constant that represents a desired value of the difference between the standing and resting heart rates, and acts as a location parameter that translates the peak of the curve of the distribution generated by the probability density function to a location representing an optimum recovery score for the subject.

$\sigma$ is the non-zero scaling constant that defines the width of the curve of the distribution generated by the probability density function.

S is a non-zero scaling constant.

The apparatus may comprise the user electronics device of the second aspect of the disclosure or the electronics module of the second aspect of the disclosure.

## Brief Description of the Drawings

Examples of the present disclosure will now be described with reference to the accompanying drawings, in which:

Figure 1 illustrates a signal trace for an ECG signal;

Figure 2 illustrates an ECG waveform that includes electrical signals for two successive heartbeats;

Figure 3 shows a schematic diagram for an example system according to aspects of the present disclosure;

Figure 4 shows a schematic diagram for an example electronics module according to aspects of the present disclosure;

Figure 5 shows a schematic diagram for another example electronics module according to aspects of the present disclosure;

Figure 6 shows a schematic diagram for an example analogue-to-digital converter used in the example electronics module of Figures 4 and 5 according to aspects of the present disclosure;

Figure 7 shows a schematic diagram of the components of an example user electronics device according to aspects of the present disclosure;

Figures 8 to 12 show screenshots of an example application running on a user electronics device according to aspects of the present disclosure;

Figure 13 shows example distributions generated for different subjects according to aspects of the present disclosure;

and

Figure 14 shows a flow diagram for an example method according to aspects of the present disclosure.

## Detailed Description

The following description with reference to the accompanying drawings is provided to assist in a comprehensive understanding of various embodiments of the disclosure as defined by the claims. It includes various specific details to assist in that understanding but these are to be regarded as merely exemplary. Accordingly, those of ordinary skill in the art will recognize that various changes and modifications of the various embodiments described herein can be made without departing from the scope of the disclosure. In addition, descriptions of well-known functions and constructions may be omitted for clarity and conciseness.

The terms and words used in the following description and claims are not limited to the bibliographical meanings but are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

It is to be understood that the singular forms "a," "an," and "the" include plural referents unless the context clearly dictates otherwise.

"Wearable article" as referred to throughout the present disclosure may refer to any form of device interface which may be worn by a user such as a smart watch, necklace, garment, bracelet, or glasses. The wearable article may be a textile article. The wearable article may be a garment. The garment may refer to an item of clothing or apparel. The garment may be a top. The top may be a shirt, t-shirt, blouse, sweater, jacket/coat, or vest. The garment may be a dress, garment brassiere, shorts, pants, arm or leg sleeve, vest, jacket/coat, glove, armband, underwear, headband, hat/cap, collar, wristband, stocking, sock, or shoe, athletic clothing, personal protective equipment, including hard hats, swimwear, wetsuit or dry suit.

The term "wearer" includes a user who is wearing, or otherwise holding, the wearable article.

The type of wearable garment may dictate the type of biosignals to be detected. For example, a hat or cap may be used to detect electroencephalogram or magnetoencephalogram signals. The wearable article/garment may be constructed from a woven or a non-woven material. The wearable article/garment may be constructed from natural fibres, synthetic fibres, or a natural fibre blended with one or more other materials which can be natural or synthetic. The yarn may be cotton. The cotton may be blended with polyester and/or viscose and/or polyamide according to the application. Silk may also be used as the natural fibre. Cellulose, wool, hemp and jute are also natural fibres that may be used in the wearable article/garment. Polyester, polycotton, nylon and viscose are synthetic fibres that may be used in the wearable article/garment.

The garment may be a tight-fitting garment. Beneficially, a tight-fitting garment helps ensure that the sensor devices of the garment are held in contact with or in the proximity of a skin surface of the wearer. The garment may be a compression garment. The garment may be an athletic garment such as an elastomeric athletic garment.

The garment has sensing units provided on an inside surface which are held in close proximity to a skin surface of a wearer wearing the garment. This enables the sensing units to measure biosignals for the wearer wearing the garment.

The sensing units may be arranged to measure one or more biosignals of a wearer wearing the garment.

"Biosignal" as referred to throughout the present disclosure may refer to signals from living beings that can be continually measured or monitored. Biosignals may be electrical or non-electrical signals. Signal variations can be time variant or spatially variant.

Sensing components may be used for measuring one or a combination of bioelectrical, bioimpedance, biochemical, biomechanical, bioacoustics, biooptical or biothermal signals of the wearer 600. The bioelectrical measurements include electrocardiograms (ECG), electrogastrograms (EGG), electroencephalograms (EEG), and electromyography (EMG). The bioimpedance measurements include plethysmography (e.g., for respiration), body composition (e.g., hydration, fat, etc.), and electro impedance tomography (EIT). The biomagnetic measurements include magnetoneurograms (MNG), magnetoencephalography (MEG), magnetogastrogram (MGG), magnetocardiogram (MCG). The biochemical measurements include glucose/lactose measurements which may be performed using chemical analysis of the wearer 600's sweat. The biomechanical measurements include blood pressure. The bioacoustics measurements include phonocardiograms (PCG). The biooptical measurements include orthopantomogram (OPG). The biothermal measurements include skin temperature and core body temperature measurements.

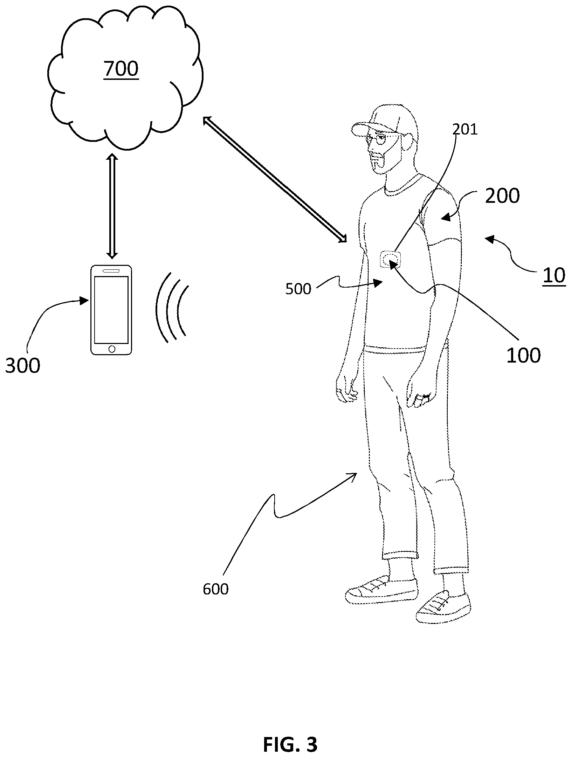

Referring to Figures 3 to 7, there is shown an example system 10 according to aspects of the present disclosure. The system 10 comprises an electronics module 100, a wearable article in the form of a garment 200, and a user electronic device 300. The garment 200 is worn by a user who in this embodiment is the wearer 600 of the garment 200.

The electronics module 100 is arranged to integrate with sensing units 400 incorporated into the garment 200 to obtain signals from the sensing units 400.

The electronics module 100 and the wearable article 200 and including the sensing units 400 comprise a wearable assembly 500.

The sensing units 400 comprise one or more sensors 209, 211 with associated conductors 203, 207 and other components and circuitry. The electronics module 100 is further arranged to wirelessly communicate data to the user electronic device 300. Various protocols enable wireless communication between the electronics module 100 and the user electronic device 300. Example communication protocols include Bluetooth ®, Bluetooth ® Low Energy, and near-field communication (NFC).

The garment 200 has an electronics module holder in the form of a pocket 201. The pocket 201 is sized to receive the electronics module 100. When disposed in the pocket 201, the electronics module 100 is arranged to receive sensor data from the sensing units 400. The electronics module 100 is therefore removable from the garment 200.

The present disclosure is not limited to electronics module holders in the form pockets.

The electronics module 100 may be configured to be releasably mechanically coupled to the garment 200. The mechanical coupling of the electronic module 100 to the garment 200 may be provided by a mechanical interface such as a clip, a plug and socket arrangement, etc. The mechanical coupling or mechanical interface may be configured to maintain the electronic module 100 in a particular orientation with respect to the garment 200 when the electronic module 100 is coupled to the garment 200. This may be beneficial in ensuring that the electronic module 100 is securely held in place with respect to the garment 200 and/or that any electronic coupling of the electronic module 100 and the garment 200 (or a component of the garment 200) can be optimized. The mechanical coupling may be maintained using friction or using a positively engaging mechanism, for example.

Beneficially, the removable electronic module 100 may contain all the components required for data transmission and processing such that the garment 200 only comprises the sensing units 400 e.g. the sensors 209, 211 and communication pathways 203, 207. In this way, manufacture of the garment 200 may be simplified. In addition, it may be easier to clean a garment 200 which has fewer electronic components attached thereto or incorporated therein. Furthermore, the removable electronic module 100 may be easier to maintain and/or troubleshoot than embedded electronics. The electronic module 100 may comprise flexible electronics such as a flexible printed circuit (FPC).

The electronic module 100 may be configured to be electrically coupled to the garment 200.

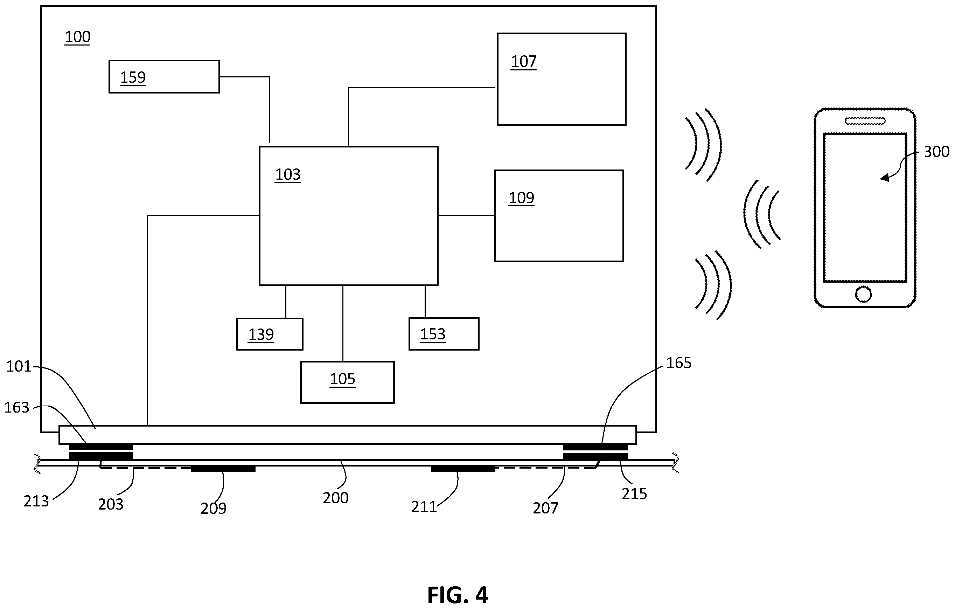

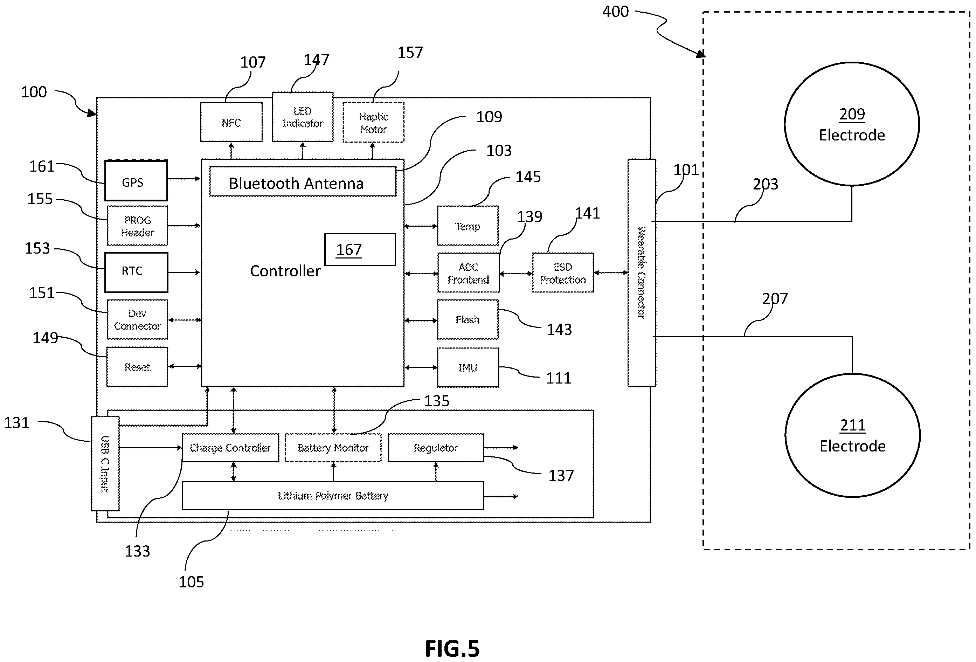

Referring to Figure 4, there is shown a schematic diagram of an example of the electronics module 100 of Figure 1. A more detailed block diagram of the electronics components of electronics module 100 and garment are shown in Figure 5.

The electronics module 100 comprises an interface 101, a controller 103, a power source 105, and one or more communication devices which, in the exemplar embodiment comprises a first antenna 107, a second antenna 109 and a wireless communicator 159. The electronics module 100 also includes an input unit such as a proximity sensor or a motion sensor 111, for example in the form of an inertial measurement unit (IMU).

The electronics module 100 also includes additional peripheral devices that are used to perform specific functions as will be described in further detail herein.

The interface 101 is arranged to communicatively couple with the sensing unit 400 of the garment 200. The sensing unit 400 comprises - in this example - the two sensors 209, 211 coupled to respective first and second electrically conductive pathways 203, 207, each with respective termination points 213, 215. The interface 101 receives signals from the sensors 209, 211. The controller 103 is communicatively coupled to the interface 101 and is arranged to receive the signals from the interface 101 for further processing.

The interface 101 of the embodiment described herein comprises first and second contacts 163, 165 which are arranged to be communicatively coupled to the termination points 213, 215 the respective first and second electrically conductive pathways 203, 207. The coupling between the termination points 213, 215 and the respective first and second contacts 163, 165 may be conductive or a wireless (e.g. inductive) communication coupling.

In this example the sensors 209, 211 are used to measure electropotential signals such as electrocardiogram (ECG) signals, although the sensors 209, 211 could be configured to measure other biosignal types as also discussed above.

In this embodiment, the sensors 209, 211 are configured for so-called dry connection to the wearer's skin to measure ECG signals.

The power source 105 may comprise a plurality of power sources. The power source 105 may be a battery. The battery may be a rechargeable battery. The battery may be a rechargeable battery adapted to be charged wirelessly such as by inductive charging. The power source 105 may comprise an energy harvesting device. The energy harvesting device may be configured to generate electric power signals in response to kinetic events such as kinetic events 10 performed by the wearer 600 of the garment 200. The kinetic event could include walking, running, exercising or respiration of the wearer 600. The energy harvesting material may comprise a piezoelectric material which generates electricity in response to mechanical deformation of the converter. The energy harvesting device may harvest energy from body heat of the wearer 600 of the garment. The energy harvesting device may be a thermoelectric energy harvesting device. The power source 105 may be a super capacitor, or an energy cell.

The first antenna 107 is arranged to communicatively couple with the user electronic device 300 using a first communication protocol. In the example described herein, the first antenna 107 is a passive or active tag such as a passive or active Radio Frequency Identification (RFID) tag or Near Field Communication (NFC) tag. These tags comprise a communication module as well as a memory which stores the information, and a radio chip. The user electronic device 300 is powered to induce a magnetic field in an antenna of the user electronic device 300. When the user electronic device 300 is placed in the magnetic field of the communication module antenna 107, the user electronic device 300 induces current in the communication module antenna 107. This induced current triggers the electronics module 100 to retrieve the information from the memory of the tag and transmit the same back to the user electronic device 300.

In an example operation, the user electronic device 300 is brought into proximity with the electronics module 100. In response to this, the electronics module 100 is configured to energize the first antenna 107 to transmit information to the user electronic device 300 over the first wireless communication protocol. Beneficially, this means that the act of the user electronic device 300 approaching the electronics module 100 energizes the first antenna 107 to transmit the information to the user electronic device 300.

The information may comprise a unique identifier for the electronics module 100. The unique identifier for the electronics module 100 may be an address for the electronics module 100 such as a MAC address or Bluetooth ® address.

The information may comprise authentication information used to facilitate the pairing between the electronics module 100 and the user electronic device 300 over the second wireless communication protocol. This means that the transmitted information is used as part of an out of band (OOB) pairing process.

The information may comprise application information which may be used by the user electronic device 300 to start an application on the user electronic device 300 or configure an application running on the user electronic device 300. The application may be started on the user electronic device 300 automatically (e.g. without wearer 600 input). Alternatively, the application information may cause the user electronic device 300 to prompt the wearer 600 to start the application on the user electronic device. The information may comprise a uniform resource identifier such as a uniform resource location to be accessed by the user electronic device, or text to be displayed on the user electronic device for example. It will be appreciated that the same electronics module 100 can transmit any of the above example information either alone or in combination. The electronics module 100 may transmit different types of information depending on the current operational state of the electronics module 100 and based on information it receives from other devices such as the user electronic device 300.

The second antenna 109 is arranged to communicatively couple with the user electronic device 300 over a second wireless communication protocol. The second wireless communication protocol may be a Bluetooth ® protocol, Bluetooth ® 5 or a Bluetooth ® Low Energy protocol but is not limited to any particular communication protocol. In the present embodiment, the second antenna 109 is integrated into controller 103. The second antenna 109 enables communication between the user electronic device 300 and the controller 100 for configuration and set up of the controller 103 and the peripheral devices as may be required. Configuration of the controller 103 and peripheral devices utilises the Bluetooth ® protocol.

The wireless communicator 159 may be an alternative, or in addition to, the first and second antennas 107, 109.

Other wireless communication protocols can also be used, such as used for communication over: a wireless wide area network (WWAN), a wireless metro area network (WMAN), a wireless local area network (WLAN), a wireless personal area network (WPAN), Bluetooth ® Low Energy, Bluetooth ® Mesh, Thread, Zigbee, IEEE 802.15.4, Ant, a Global Navigation Satellite System (GNSS), a cellular communication network, or any other electromagnetic RF communication protocol. The cellular communication network may be a fourth generation (4G) LTE, LTE Advanced (LTE-A), LTE Cat-M1, LTE Cat-M2, NB-IoT, fifth generation (5G), sixth generation (6G), and/or any other present or future developed cellular wireless network.

The electronics module 100 includes a clock unit in the form of a real time clock (RTC) 153 coupled to the controller 103 and, for example, to be used for data logging, clock building, time stamping, timers, and alarms. As an example, the RTC 153 is driven by a low frequency clock source or crystal operated at 32.768 Hz.

The electronics module 100 also includes a location device 161 such as a GNSS (Global Navigation Satellite System) device which is arranged to provide location and position data for applications as required. In particular, the location device 161 provides geographical location data at least to a nation state level. Any device suitable for providing location, navigation or for tracking the position could be utilised. The GNSS device may include device may include Global Positioning System (GPS), BeiDou Navigation Satellite System (BDS) and the Galileo system devices.

The power source 105 in this example is a lithium polymer battery 105. The battery 105 is rechargeable and charged via a USB C input 131 of the electronics module 100. Of course, the present disclosure is not limited to recharging via USB and instead other forms of charging such as inductive of far field wireless charging are within the scope of the present disclosure. Additional battery management functionality is provided in terms of a charge controller 133, battery monitor 135 and regulator 147. These components may be provided through use of a 30 dedicated power management integrated circuit (PMIC).

The USB C input 131 is also coupled to the controller 131 to enable direct communication with the controller 103 with an external device if required.

The controller 103 is communicatively connected to a battery monitor 135 so that that the controller 103 may obtain information about the state of charge of the battery 105.

The controller 103 has an internal memory 167 and is also communicatively connected to an external memory 143 which in this example is a NAND Flash memory. The memory 143 is used to for the storage of data when no wireless connection is available between the electronics module 100 and a user electronic device 300. The memory 143 may have a storage capacity of at least 1GB and preferably at least 2 GB.

The electronics module 100 also comprises a temperature sensor 145 and a light emitting diode 147 for conveying status information. The electronic module 100 also comprises conventional electronics components including a power-on-reset generator 149, a development connector 151, the real time clock 153 and a PROG header 155.

Additionally, the electronics module 100 may comprise a haptic feedback unit 157 for providing a haptic (vibrational) feedback to the wearer 600.

The wireless communicator 159 may provide wireless communication capabilities for the garment 200 and enables the garment to communicate via one or more wireless communication protocols to a remote server 700. Wireless communications may include : a wireless wide area network (WWAN), a wireless metro area network (WMAN), a wireless local area network (WLAN), a wireless personal area network (WPAN), Bluetooth ® Low Energy, Bluetooth ® Mesh, Bluetooth ® 5, Thread, Zigbee, IEEE 802.15.4, Ant, a near field communication (NFC), a Global Navigation Satellite System (GNSS), a cellular communication network, or any other electromagnetic RF communication protocol. The cellular communication network may be a fourth generation (4G) LTE, LTE Advanced (LTE-A), LTE Cat-M1, LTE Cat-M2, NB-IoT, fifth generation (5G), sixth generation (6G), and/or any other present or future developed cellular wireless network.

The electronics module 100 may additionally comprise a Universal Integrated Circuit Card (UICC) that enables the garment to access services provided by a mobile network operator (MNO) or virtual mobile network operator (VMNO). The UICC may include at least a read-only memory (ROM) configured to store an MNO or VMNO profile that the garment can utilize to register and interact with an MNO or VMNO. The UICC may be in the form of a Subscriber Identity Module (SIM) card. The electronics module 100 may have a receiving section arranged to receive the SIM card. In other examples, the UICC is embedded directly into a controller of the electronics module 100. That is, the UICC may be an electronic/embedded UICC (eUICC). A eUICC is beneficial as it removes the need to store a number of MNO profiles, i.e. electronic Subscriber Identity Modules (eSIMs). Moreover, eSIMs can be remotely provisioned to garments. The electronics module 100 may comprise a secure element that represents an 35 embedded Universal Integrated Circuit Card (eUICC). In the present disclosure, the electronics module may also be referred to as an electronics device or unit. These terms may be used interchangeably.

The controller 103 is connected to the interface 101 via an analog-to-digital converter (ADC) front end 139 and an electrostatic discharge (ESD) protection circuit 141.

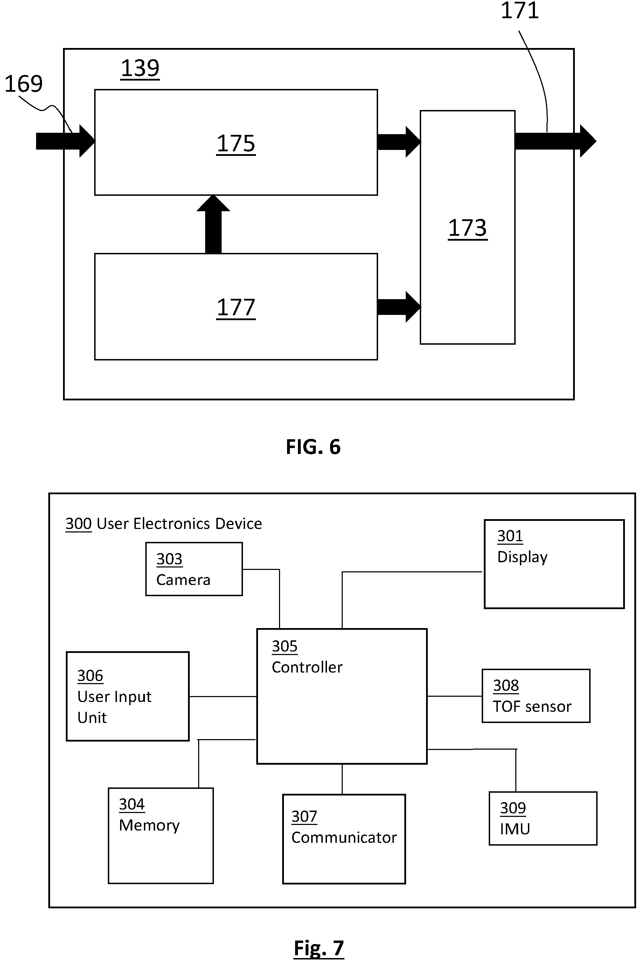

Figure 6 is a schematic illustration of the component circuitry for the ADC front end 139.

In the example described herein, the ADC front end 139 is an integrated circuit (IC) chip which converts the raw analogue biosignal received from the sensors 209, 211 into a digital signal for further processing by the controller 103. ADC IC chips are known, and any suitable one can be utilised to provide this functionality. ADC IC chips for ECG applications include, for example, the MAX30003 chip produced by Maxim Integrated Products Inc.

The ADC front end 139 includes an input 169 and an output 171.

Raw biosignals from the electrodes 209, 211 are input to the ADC front end 139, where received signals are processed in an ECG channel 175 and subject to appropriate filtering through high pass and low pass filters for static discharge and interference reduction as well as for reducing bandwidth prior to conversion to digital signals. The reduction in bandwidth is important to remove or reduce motion artefacts that give rise to noise in the signal due to movement of the sensors 209, 211.

The output digital signals may be decimated to reduce the sampling rate prior to being passed to a serial programmable interface (SPI) 173 of the ADC front end 139.

ADC front end IC chips suitable for ECG applications may be configured to determine information from the input biosignals such as heart rate and the QRS complex and including the R-R interval. Support circuitry 177 provides base voltages for the ECG channel 175.

The determining of the QRS complex can be implemented for example using the known Pan Tomkins algorithm as described in Pan, Jiapu; Tompkins, Willis J. (March 1985). "A Real-Time QRS Detection Algorithm". IEEE Transactions on Biomedical Engineering. BME-32 (3): 230-236.

Signals are output to the controller 103 via the SPI 173.

The controller 103 can also be configured to apply digital signal processing (DSP) to the digital signal from the ADC front end 139.

The DSP may include noise filtering additional to that carried out in the ADC front end 139 and may also include additional processing to determine further information about the signal from the ADC front end 139.

The controller 103 is configured to send the biosignals to the user electronic device 300 using either of the first antenna 107, second antenna 109, or wireless communicator 159. The biosignals sent to the user electronic device 300 in this example comprise the inter beat interval (IBI) values representing the time differences between successive R peaks in the measured ECG signal.

The user electronic device 300 in the example of Figure 7 is in the form of a mobile phone or tablet and comprises a controller 305, a memory 304, a wireless communicator 307, a display 301, a user input unit 306, a capturing device in the form of a camera 303 and an inertial measurement unit (IMU) 309. The controller 305 provides overall control to the user electronic device 300.

The user input unit 306 receives inputs from the user such as a user credential.

The memory 304 stores information for the user electronic device 300.

The display 301 is arranged to display a user interface for applications operable on the user electronic device 300.

The IMU 309 provides motion and/or orientation detection and may comprise an accelerometer and optionally one or both of a gyroscope and a magnetometer.

The user electronic device 300 may also include a biometric sensor. The biometric sensor may be used to identify a user or users of device based on unique physiological features. The biometric sensor may be: a fingerprint sensor used to capture an image of a user's fingerprint; an iris scanner or a retina scanner configured to capture an image of a user's iris or retina; an ECG module used to measure the user's ECG; or the camera of the user electronic arranged to capture the face of the user. The biometric sensor may be an internal module of the user electronic device. The biometric module may be an external (stand-alone) device which may be coupled to the user electronic device by a wired or wireless link.

The controller 305 is configured to launch an application which is configured to display insights derived from the biosignal data processed by the ADC front end 139 of the electronics module 100, input to electronics module controller 103, and then transmitted from the electronics module 100. The transmitted data is received by the wireless communicator 307 of the user electronic device 300 and input to the controller 305.

Insights include, but are not limited to, an ECG signal trace i.e. the QRS complex, heart rate, respiration rate, core temperature but can also include identification data for the wearer 600 using the wearable assembly 500.

The display 301 may be a presence-sensitive display and therefore may comprise the user input unit 306. The presence-sensitive display may include a display component and a presence-sensitive input component. The presence sensitive display may be a touch-screen display arranged as part of the user interface.

The user electronics device may also comprise a time of flight (TOF) sensor 308.

User electronic devices in accordance with the present invention are not limited to mobile phones or tablets and may take the form of any electronic device which may be used by a user to perform the methods according to aspects of the present invention. The user electronic device 300 may be a electronics module such as a smartphone, tablet personal computer (PC), mobile phone, smart phone, video telephone, laptop PC, netbook computer, personal digital assistant (PDA), mobile medical device, camera or wearable device. The user electronic device 300 may include a head-mounted device such as an Augmented Reality, Virtual Reality or Mixed Reality head-mounted device. The user electronic device 300 may be desktop PC, workstations, television apparatus or a projector, e.g. arranged to project a display onto a surface.

In use, the electronics module 100 is configured to receive raw biosignal data from the sensors 209, 211 and which are coupled to the controller 103 via the interface 101 and the ADC front end 139 for further processing and transmission to the user electronic device 300 as described above. The data transmitted to the user electronics device 300 includes raw or processed biosignal data such as ECG data, heart rate, respiration data, core temperature and other insights as determined.

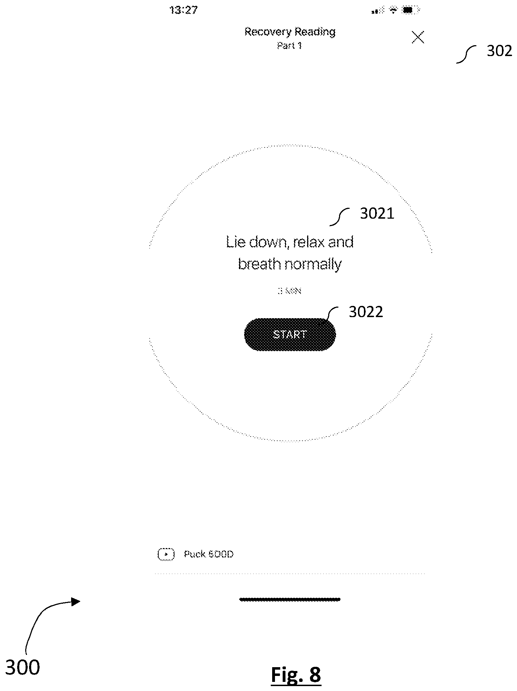

The controller 305 of the user electronics device 300 is also operable to launch an application which is configured to determine and output (e.g. display) a recovery score for the subject. The user interface 302 displayed by the user electronics device 300 during the recovery score test is shown in Figures 8 to 12.

Referring to Figure 8, the recovery score application displays on the user interface 302 a prompt to the user to lie down, relax and breath normally. This instructs the user to adopt a resting position. The user then presses the start button 3022 to being the measurement procedure.

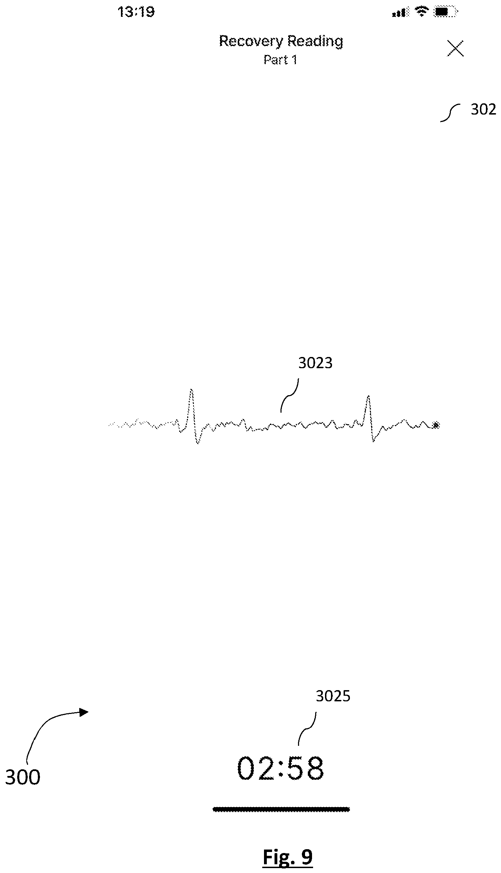

After the user presses the start button 3022, the recovery score application transitions to the screen shown in Figure 9. The recovery score application displays an ECG waveform 3023 using biosignal data transmitted by the electronics module 100 to the user electronics device 300. The biosignal data received by the user electronic device 300 includes the recorded ECG data. The recovery score application records heartbeat data while the user is in the resting position for three minutes (other time ranges are possible) and thus obtains a first sequence of heartbeat data samples of the subject while in the resting position. A timer 3025 displays the remaining time to the user.

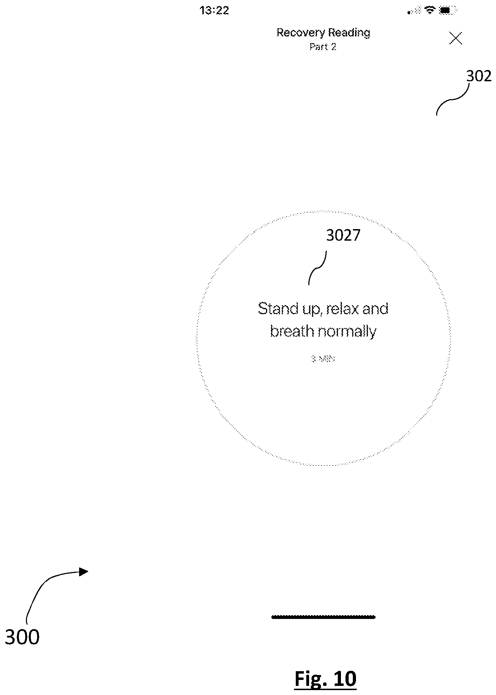

After the three-minute timer has elapsed, the recovery score application transitions to the screen shown in Figure 10. The user interface 302 displays a prompt 3027 to the user to stand up, relax and breathe normally. This instructs the user to adopt the standing position. In addition to a visual notification, audible and/or haptic feedback may prompt the user to adopt the standing position.

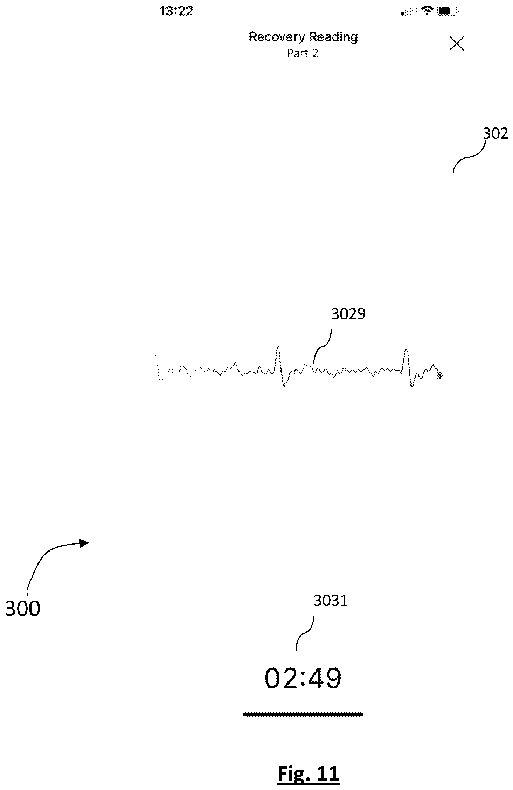

After a predetermined time interval of generally a few seconds, the recovery score application transitions to the screen in Figure 11. The recovery score application displays an ECG waveform 3029 using biosignal data transmitted by the electronics module 100 to the user electronics device 300. The biosignal data received by the user electronic device 300 includes the recorded ECG data. The recovery score application records heartbeat data while the user is in the standing position for three minutes (other time ranges are possible) obtain a second sequence of heartbeat data samples of the subject while in the standing position. A timer 3031 displays the remaining time to the user.

Generally, the first and second sequence of heartbeat data samples comprise inter beat interval (IBI) values that are extracted from the ECG signals. That is, the user electronics device 300 receives the extracted IBI values from the electronics module 100. In other examples, the user electronics device 300 may extract the IBI values from the received ECG signals.

After the timer has elapsed and the three minutes of heartbeat data has been obtained while the subject is in the standing position, the recovery score application calculates a recovery score for the subject.

In particular, the resting heart rate $HR_{rest}$ is calculated from the first sequence of heartbeat data samples. This involves calculating the average (e.g. mean) IBI value in milliseconds for the first sequence of heartbeat data samples and dividing 60000 by the average IBI value.

Further, the standing heartrate $HR_{stand}$ is calculated from the second sequence of heartbeat data samples. This involves calculating the average (e.g. mean) IBI value in milliseconds for the second sequence of heartbeat data samples and by dividing 60000 by the average IBI values.

Not all of the recorded heartbeat data samples may be used in calculating the resting and standing heartrates. For example, the first one minute of heartbeat data samples recorded when the user is standing may be disregarded.

The resting and standing heart rates are then input into a probability density function which generates, as an output, a recovery score for the subject. The recovery score provides a value between 0 (indicating a very low recovery score) and a maximum value (e.g. 10) indicating an optimum recovery score.

The probability density function used by the recovery score application is of the form:

Equation 1:

$$f(HR_{rest}, HR_{stand}) = S \times \frac{A}{\sigma} \times B^{\left(\frac{(HR_{stand} - HR_{rest}) - \mu}{\sigma}\right)^2}$$

A is a non-zero scaling constant.

A may be equal to $\left(\frac{1}{\sqrt{2\pi}}\right)$.

Preferably, the probability density function is scaled such that the probability density function outputs a score between 0 and a pre-defined, and preferably intuitive, maximum value (e.g. 10). In these examples, S acts as a scaling constant to set the maximum value of the function.

If the desired maximum value = 10 then S can be determined as being equal to: $S = 10\sigma\sqrt{2\pi}$

Or more simply, $S \times \frac{A}{\sigma} = 10$

The score does not have to be between 0 and 10. The score may be between any values as desired by the skilled person and which can be determined by appropriately selecting the value of the scaling constant S. That is, S may be an integer which sets the maximum desired value for the recovery score.

More generally, S may be set such $= P \times \sigma / A$. P is a non-zero constant that sets the upper limit of the recovery score. In some examples, P = 10. P is not limited to any particular value. P may be, for example, 5, 20, 50, or 100.

In some examples, S may equal 1 such that the probability density function is not scaled.

B is a non-zero constant. In some examples, $B = e^{-\frac{1}{2}}$.

$\mu$ is a non-zero constant that represents a desired value of the difference between the standing and resting heart rates. $\mu$ is a location parameter that determines the location of the peak of the normal distribution. The peak of the normal distribution is the optimum difference between the standing and resting heart rates for the subject which represents the optimum recovery score. That is, $\mu$ translates the peak of the curve of the distribution generated by the probability density function to a location representing an optimum recovery score for the subject.

$\sigma$ is a non-zero scaling constant that defines the width of the curve of the distribution generated by the probability density function; and

In preferred examples, $\mu$ is set to be proportional to the standard deviation. In particular, preferred examples, $\mu = 3\sigma$. This centres the peak of the distribution at a position that is 3 standard deviations from a minimum expected difference between the standing and resting heart rates for the subject, and also 3 standard deviations away from a maximum expected difference between the standing and resting heart rates for the subject.

In preferred examples, $\sigma$ is determined according to the maximum expected difference between standing and resting heart rates for the subject, $OHR_{max}$, and the minimum expected difference between standing and resting heart rates for the subject, $OHR_{min}$. Here, $OHR_{max} = HR_{stand,max} - HR_{rest,min}$. Here, $OHR_{min} = HR_{stand,min} - HR_{rest,max}$.

In particular, preferred examples $\sigma$ is determined by calculating $(OHR_{max} - OHR_{min})/ C$, where C is a non-zero constant that represents the number of standard deviations required to get from $OHR_{min}$ to $\mu$ and from $\mu$ to $OHR_{max}$. C is a number greater than 0. Preferably, C = 6.

The present disclosure is not limited to any particular value of $\sigma$. This value is generally subject specific. $\sigma$ may be between 1 and 30, preferably between 1 and 25, preferably still between 1 and 20, preferably still between 1 and 15, preferably still between 1 and 10, preferably still between 1 and 5. In most preferred example $\sigma = 5$.

The present disclosure is not limited to any particular value of $OHR_{max}$ and $OHR_{min}$. These values are generally subject specific. However, in some examples, the difference between $OHR_{max}$ and $OHR_{min}$ is between 20 and 50, preferably still between 25 and 40, and preferably still is 30. In one example, $OHR_{max} = 30$ and $OHR_{min} = 0$.

The present disclosure is not limited to any particular value of $\mu$. The value of $\mu$ is proportional to the value of $\sigma$ and so will vary as $\sigma$ varies. In some examples, $\mu$ is between 5 and 25. $\mu$ is between 10 and 20. $\mu$ is 15.

In preferred examples, the function $f(HR_{rest}, HR_{stand})$ is of the form:

Equation 2:

$$f(HR_{rest}, HR_{stand}) = S \times \frac{A}{\sigma} \times B^{\left(\frac{(HR_{stand}-HR_{rest})-3\sigma}{\sigma}\right)^2}$$

In preferred examples, the function $f(HR_{rest}, HR_{stand})$ is of the form:

Equation 3:

$$f(HR_{rest}, HR_{stand}) = P \times e^{-\frac{1}{2}\left(\frac{(HR_{stand}-HR_{rest})-3\sigma}{\sigma}\right)^2}$$

**In this example,** $S = P \times \sigma \times \sqrt{2\pi}, A = 1/\sqrt{2\pi}$ and $B = e^{-\frac{1}{2}}$.

In preferred examples, the function $f(HR_{rest}, HR_{stand})$ is of the form:

Equation 4:

$$f(HR_{rest}, HR_{stand}) = 10 \times e^{-\frac{1}{2}\left(\frac{(HR_{stand}-HR_{rest})-3\sigma}{\sigma}\right)^2}$$

In this example, $S = 10 \times \sigma \times \sqrt{2\pi}, A = 1/\sqrt{2\pi}$ and $B = e^{-\frac{1}{2}}$.

In preferred examples still, $\sigma = 5$, $OHR_{max} = 30$, $OHR_{min} = 0$, $\mu = 15$. That is:

Equation 5:

$$f(HR_{rest}, HR_{stand}) = 10 \times e^{-\frac{1}{2}\left(\frac{(HR_{stand}-HR_{rest})-15}{5}\right)^2}$$

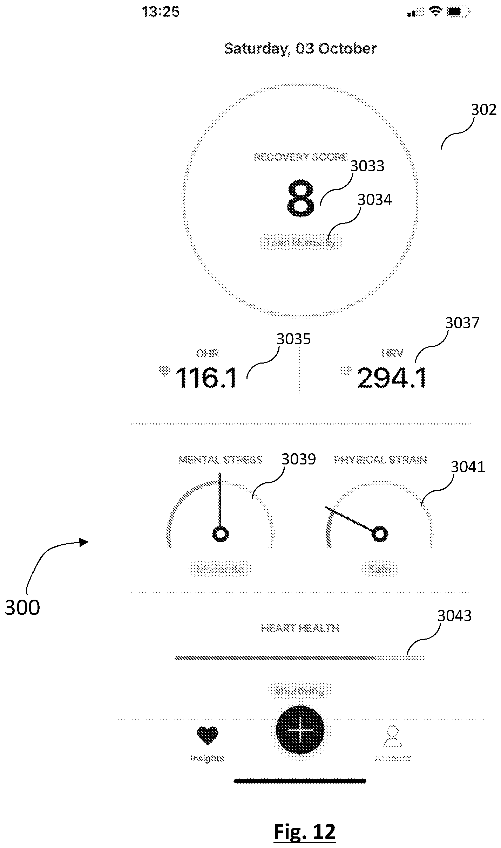

The generated recovery score is displayed to the user as shown in Figure 12. In particular a numerical value 3033 between 0 and 10 is displayed to the user to indicate the recovery score. In addition, a text box 3034 is displayed which gives a recommendation to the user based on their recovery score and potentially other factors such as hear rate variability, mental stress and physical strain. In this example, the subject has a recovery score of 8 out of 10 and is recommended to train normally.

Additional metrics are displayed to the user on the screen including the subject's orthostatic (standing) heart rate 3035, heart rate variability, 3037, mental stress score 3039, physical strain 3041 and heart health score 3041.

In some examples, recovery score corresponds to following:

| Recovery score | User Prompt |
|----------------|-------------|
| 8-10 | Train Normally |
| 5-7 | Reduce Intensity |

(continued)

| Recovery score | User Prompt |
| --- | --- |
| 2-4 | Recovery Day |
| 0-1 | Seek Medical Advice |

Additional parameters may also be considered in providing recommendation to user including other factors such as the subject's heart rate variability, mental stress and physical strain. That is, a subject with a high recovery score may still be advised to reduce their training intensity based on other parameters.

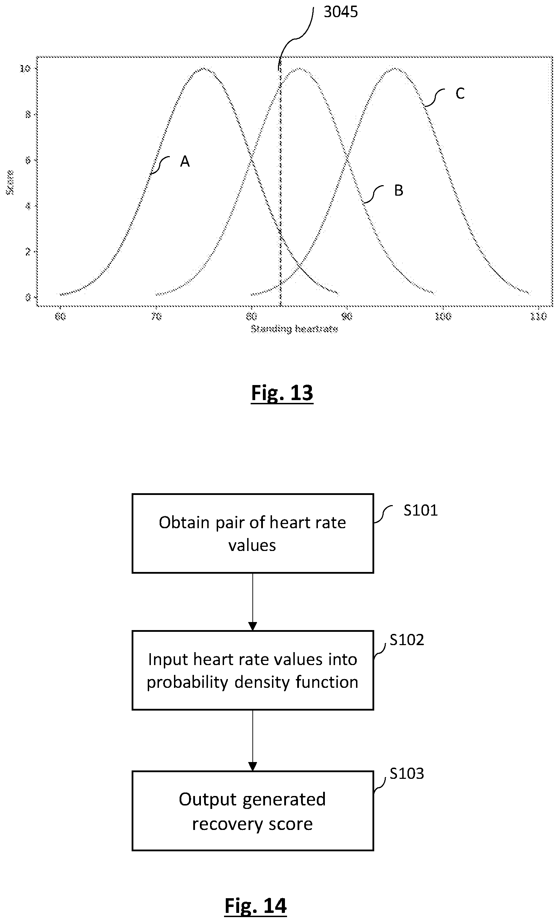

Figure 13 shows example normal distributions generated using the probability density function of Equation 5 for three different subject A, B, C. The normal distributions are generated using a fixed resting heart rate that is different for each subject and a plurality of different standing heart rates that vary over a range.

Subject A has a resting heart rate of 60.

Subject B has a resting heart rate of 70.

Subject C has a resting heart rate of 80.

It will be appreciated that different standing heart rates will give different recovery scores for each of the users. Figure 13 shows a dashed line 3045 that represents a standing heart rate value of 83 and how it intersects with the normal distributions for the three different subject's A, B and C.

If subject A has a resting heart rate of 60 and a standing heart rate of 83 then they will have a recovery score of 2.78.

If subject B has a resting heart rate of 70 and a standing heart rate of 83 then they will have a recovery score of 9.23.

If subject B has a resting heart rate of 80 and a standing heart rate of 83 then they will have a recovery score of 0.56.

Figure 14 shows a process flow diagram for an example method of calculating a recovery score according to aspects of the present disclosure.

Step S101 comprises obtaining a pair of heartrate values, a first of the values, $HR_{rest}$, representing the heartrate of the subject in a resting position and a second of the values, $HR_{stand}$, representing the heartrate of the subject in a standing position;

Step S102 comprises inputting the pair of heartrate values into a probability density function $f(HR_{rest}, HR_{stand})$ which generates as an output a value representing a recovery score for the subject, wherein the function $f(HR_{rest}, HR_{stand})$ is of the form shown in Equation 1 above.

Step S103 comprises outputting the generated recovery score.

In summary, there is provided a method and system of calculating a recovery score for a subject. The method comprises obtaining a pair of heartrate values, a first of the values, $HR_{rest}$, representing the heartrate of the subject in a resting position and a second of the values, $HR_{stand}$, representing the heartrate of the subject in a standing position (S101). The method further comprises inputting the pair of heartrate values into a probability density function $f(HR_{rest}, HR_{stand})$ which generates as an output a value representing a recovery score for the subject (S102). The method further comprises outputting the generated recovery score (S103).

In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables.

Although the example embodiments have been described with reference to the components, modules and units discussed herein, such functional elements may be combined into fewer elements or separated into additional elements. Various combinations of optional features have been described herein, and it will be appreciated that described features may be combined in any suitable combination. In particular, the features of any one example embodiment may be combined with features of any other embodiment, as appropriate, except where such combinations are mutually exclusive. Throughout this specification, the term "comprising" or "comprises" means including the component(s) specified but not to the exclusion of the presence of others.

All of the features disclosed in this specification (including any accompanying claims, abstract and drawings), and/or all of the steps of any method or process so disclosed, may be combined in any combination, except combinations where at least some of such features and/or steps are mutually exclusive.

**Claims**

1. A computer-implemented method of calculating a recovery score for a subject, the method comprising:

    obtaining a pair of heartrate values, a first of the values, $HR_{rest}$, representing the heartrate of the subject in a resting position and a second of the values, $HR_{stand}$, representing the heartrate of the subject in a standing position (S101);
    **characterised in that** the method further comprises:

    inputting the pair of heartrate values into a probability density function $f(HR_{rest}, HR_{stand})$ which uses the difference between the value of $(HR_{stand} - HR_{rest})$ and a non-zero constant, $\mu$, that represents a desired value of the difference between the standing and resting heart rates, to generate a recovery score for the subject (S102), wherein $f(HR_{rest}, HR_{stand})$ is of the form:

$$f(HR_{rest}, HR_{stand}) = S \times \frac{A}{\sigma} \times B^{\left(\frac{(HR_{stand} - HR_{rest}) - \mu}{\sigma}\right)^2},$$

    wherein A and B are non-zero constants,
    wherein $\mu$ is a non-zero constant that represents a desired value of the difference between the standing and resting heart rates, and acts as a location parameter that translates the peak of the curve of the distribution generated by the probability density function to a location representing an optimum recovery score for the subject,
    wherein S is a non-zero scaling constant, and
    wherein $\sigma$ is a non-zero scaling constant that defines the width of the curve of the distribution generated by the probability density function; and
    outputting the generated recovery score (S103).

2. A computer-implemented method as claimed in claim 1, wherein $\mu$ is proportional to $\sigma$, optionally wherein $\mu = 3\sigma$.

3. A computer-implemented method as claimed in any of claims 1 to 2, wherein $\sigma$ is determined according to the maximum expected difference between standing and resting heart rates for the subject, $OHR_{max}$, and the minimum expected difference between standing and resting heart rates for the subject, $OHR_{min}$.

4. A computer-implemented method as claimed in claim 3, wherein $\sigma$ is determined by calculating $OHR_{max} - OHR_{min}$,

5. A computer-implemented method as claimed in claim 4, wherein $\sigma$ is determined by dividing $OHR_{max} - OHR_{min}$ by a non-zero constant C, optionally wherein C represents the number of standard deviations required to get from $OHR_{min}$ to $\mu$ and from $\mu$ to $OHR_{max}$, optionally wherein C = 6.

6. A computer-implemented method as claimed in any of claims 3 to 5, wherein the difference between $OHR_{max}$ and $OHR_{min}$ is between 20 and 50.

7. A computer-implemented method as claimed in claim 6, wherein the difference between $OHR_{max}$ and $OHR_{min}$ is between 25 and 40, optionally wherein the difference between $OHR_{max}$ and $OHR_{min}$ is 30, and optionally wherein $OHR_{max} = 30$ and $OHR_{min} = 0$.

8. A computer-implemented method as claimed in any preceding claim, wherein $B = e^{-\frac{1}{2}}$

9. A computer-implemented method as claimed in any preceding claim, wherein $\mu = 3\sigma$, $\sigma = 5$, and optionally wherein $S \times \frac{A}{\sigma} = 10$ .

10. A computer-implemented method as claimed in any preceding claim, wherein $S = P \times \frac{\sigma}{A}$ wherein P is a non-zero constant that sets the upper limit of the recovery score, optionally wherein $A = 1/\sqrt{2\pi}$ , and wherein

$$S = P \times \sigma \times \sqrt{2\pi}$$

11. A computer-implemented method as claimed in any preceding claim, wherein $\mu$ is between 5 and 25, optionally wherein $\mu$ is between 10 and 20, and optionally wherein $\mu$ is 15.

12. A computer-implemented method as claimed in any preceding claim, wherein obtaining the pair of heartrate values comprises:

   obtaining a first sequence of heartbeat data samples of the subject in a resting position and a second sequence of heartbeat data samples of the subject in a standing position, wherein the heartbeat data samples comprises inter-beat interval, IBI, values representing the time between successive heartbeats;
   calculating $HR_{rest}$ from the first sequence of heartbeat data samples; and
   calculating $HR_{stand}$ from the second sequence of heartbeat data samples.

13. A computer-implemented method as claimed in claim 12, wherein calculating $HR_{rest}$ comprises dividing 60000 by the average IBI value in milliseconds for the first sequence of heartbeat data samples, and wherein calculating $HR_{stand}$ comprises dividing 60000 by the average IBI value in milliseconds for the second sequence of heartbeat data samples.

14. A computer-readable medium having instructions recorded thereon which, when executed by a processor, cause the processor to perform the method as claimed in any preceding claim.

15. A system for calculating a recovery score for a subject, the system comprising a processor and a memory, the memory storing instructions which when executed by the processor cause the processor to perform operations comprising:

   obtaining a pair of heartrate values, a first of the values, $HR_{rest}$, representing the heartrate of the user in a resting position and a second of the values, $HR_{stand}$, representing the heartrate of the user in a standing position (S101);
   **characterised in that** the operations further comprise:

   inputting the pair of heartrate values into a probability density function $f(HR_{rest}, HR_{stand})$ which uses the difference between ($HR_{stand}$ - $HR_{rest}$) and a non-zero constant, $\mu$, that represents a desired value of the difference between the standing and resting heart rates, to generate a recovery score for the subject (S102), wherein $f(HR_{rest}, HR_{stand})$ is of the form:

$$f(HR_{rest}, HR_{stand}) = S \times \frac{A}{\sigma} \times B^{\left(\frac{(HR_{stand} - HR_{rest}) - \mu}{\sigma}\right)^2},$$

   wherein A and B are non-zero constants,
   wherein $\mu$ is a non-zero constant that represents a desired value of the difference between the standing and resting heart rates, and acts as a location parameter that translates the peak of the curve of the distribution generated by the probability density function to a location representing an optimum recovery score for the subject,
   wherein S is a non-zero scaling constant, and

   wherein σ is a non-zero scaling constant that defines the width of the curve of the distribution generated by the probability density function; and
   outputting the generated recovery score (S103).

**Patentansprüche**

1. Computerimplementiertes Verfahren zum Berechnen einer Wiederherstellungsbewertung für ein Subjekt, wobei das Verfahren umfasst:

   Erhalten eines Paares von Herzfrequenzwerten, wobei ein erster der Werte, $HR_{ruhend}$, die Herzfrequenz des Subjekts in einer ruhenden Position darstellt und ein zweiter der Werte, $HR_{stehend}$, die Herzfrequenz des Subjekts in einer stehenden Position (S101) darstellt;

**dadurch gekennzeichnet, dass** das Verfahren ferner umfasst:

Eingeben des Paares von Herzfrequenzwerten in eine Wahrscheinlichkeitsdichtefunktion $f(HR_{ruhend},$ $HR_{stehend})$, welche die Differenz zwischen dem Wert von $(HR_{stehend} - HR_{ruhend})$ und einer Konstante ungleich Null, $\mu$ verwendet, die einen gewünschten Wert der Differenz zwischen der stehenden und der ruhenden Herzfrequenz darstellt, um eine Wiederherstellungsbewertung für das Subjekt (S102) zu generieren, wobei $f(HR_{ruhend}, HR_{stehend})$ diese Form hat:

$$f(HR_{ruhend}, HR_{stehend}) = S \times \frac{A}{\sigma} \times B \left( \frac{(HR_{stehend} - HR_{ruhend}) - \mu}{\sigma} \right)^2,$$

wobei A und B Konstanten ungleich Null sind,
wobei $\mu$ eine Konstante ungleich Null ist, die einen gewünschten Wert der Differenz zwischen der stehenden und der ruhenden Herzfrequenz darstellt und als Ortsparameter fungiert, der die Spitze der Kurve der Verteilung, die durch die Wahrscheinlichkeitsdichtefunktion generiert wird, in einen Ort übersetzt, der eine optimale Wiederherstellungsbewertung für das Subjekt darstellt,
wobei S eine Skalierungskonstante ungleich Null ist und wobei $\sigma$ eine Skalierungskonstante ungleich Null ist, welche die Weite der Kurve der Verteilung definiert, die durch die Wahrscheinlichkeitsdichtefunktion generiert wird; und
Ausgeben der generierten Wiederherstellungsbewertung (S103).

2. Computerimplementiertes Verfahren nach Anspruch 1, wobei $\mu$ proportional zu $\sigma$ ist, wobei optional $\mu = 3\sigma$ ist.

3. Computerimplementiertes Verfahren nach einem der Ansprüche 1 bis 2, wobei $\sigma$ gemäß der maximalen erwarteten Differenz zwischen der stehenden und der ruhenden Herzfrequenz für das Subjekt, $OHR_{max}$, und der minimalen erwarteten Differenz zwischen der stehenden und der ruhenden Herzfrequenz für das Subjekt, $OHR_{min}$ bestimmt wird.

4. Computerimplementiertes Verfahren nach Anspruch 3, wobei $\sigma$ durch Berechnen von $OHR_{max} - OHR_{min}$ bestimmt wird.

5. Computerimplementiertes Verfahren nach Anspruch 4, wobei $\sigma$ durch Teilen von $OHR_{max} - OHR_{min}$ durch eine Konstante C ungleich Null bestimmt wird, wobei optional C die Anzahl von Standardabweichungen darstellt, die erforderlich sind, um von $OHR_{min}$ zu $\mu$ und von $\mu$ zu $OHR_{max}$ zu gelangen, wobei optional C = 6 ist.

6. Computerimplementiertes Verfahren nach einem der Ansprüche 3 bis 5, wobei die Differenz zwischen $OHR_{max}$ und $OHR_{min}$ zwischen 20 und 50 liegt.

7. Computerimplementiertes Verfahren nach Anspruch 6, wobei die Differenz zwischen $OHR_{max}$ und $OHR_{min}$ zwischen 25 und 40 liegt, wobei optional die Differenz zwischen $OHR_{max}$ und $OHR_{min}$ 30 beträgt und wobei optional $OHR_{max} = 30$ ist und $OHR_{min} = 0$ ist.

8. Computerimplementiertes Verfahren nach einem der vorstehenden Ansprüche, wobei $B = e^{-\frac{1}{2}}$

9. Computerimplementiertes Verfahren nach einem der vorstehenden Ansprüche, wobei $\mu = 3\sigma$ ist, $\sigma = 5$ ist und wobei optional $S \times \frac{A}{\sigma} = 10$ ist.

10. Computerimplementiertes Verfahren nach einem der vorstehenden Ansprüche, wobei $S = P \times \frac{\sigma}{A}$ ist, wobei P eine Konstante ungleich Null ist, die den oberen Grenzwert der Wiederherstellungsbewertung festlegt, wobei optional $A = 1/\sqrt{2\pi}$ ist und wobei $S = P \times \sigma \times \sqrt{2\pi}$ ist.

11. Computerimplementiertes Verfahren nach einem der vorstehenden Ansprüche, wobei $\mu$ zwischen 5 und 25 liegt,

wobei optional $\mu$ zwischen 10 und 20 liegt und wobei optional $\mu$ gleich 15 ist.

12. Computerimplementiertes Verfahren nach einem der vorstehenden Ansprüche, wobei das Erhalten des Paares von Herzfrequenzwerten umfasst:

Erhalten einer ersten Sequenz von Herzfrequenz-Datenproben des Subjekts in einer ruhenden Position und einer zweiten Sequenz von Herzfrequenz-Datenproben des Subjekts in einer stehenden Position, wobei die Herzfrequenz-Datenproben Zwischenschlagsintervall-Werte, IBI-Werte, umfassen, welche die Zeit zwischen aufeinanderfolgenden Herzschlägen darstellen;
Berechnen von $HR_{ruhend}$ anhand der ersten Sequenz von Herzfrequenz-Datenproben; und
Berechnen von $HR_{stehend}$ anhand der zweiten Sequenz von Herzfrequenz-Datenproben.

13. Computerimplementiertes Verfahren nach Anspruch 12, wobei das Berechnen von $HR_{ruhend}$ das Teilen von 60000 durch den IBI-Durchschnittswert in Millisekunden für die erste Sequenz von Herzfrequenz-Datenproben umfasst, und wobei das Berechnen von $HR_{stehend}$ das Teilen von 60000 durch den IBI-Durchschnittswert in Millisekunden für die zweite Sequenz von Herzfrequenz-Datenproben umfasst.

14. Computerlesbares Medium, auf dem Anweisungen gespeichert sind, die, wenn sie von einem Prozessor ausgeführt werden, den Prozessor veranlassen, das Verfahren nach einem der vorstehenden Ansprüche durchzuführen.

15. System zum Berechnen einer Wiederherstellungsbewertung für ein Subjekt, wobei das System einen Prozessor und einen Speicher umfasst, wobei der Speicher Anweisungen speichert, die, wenn sie von dem Prozessor ausgeführt werden, den Prozessor veranlassen, Operationen durchzuführen, die dies umfassen:

Erhalten eines Paares von Herzfrequenzwerten, wobei ein erster der Werte, $HR_{ruhend}$, die Herzfrequenz des Benutzers in einer ruhenden Position darstellt und ein zweiter der Werte, $HR_{stehend}$, die Herzfrequenz des Benutzers in einer stehenden Position (S101) darstellt;
**dadurch gekennzeichnet, dass** die Operationen ferner umfassen:

Eingeben des Paares von Herzfrequenzwerten in eine Wahrscheinlichkeitsdichtefunktion $f(HR_{ruhend}, HR_{stehend})$, welche die Differenz zwischen ($HR_{stehend}$ - $HR_{ruhend}$) und eine Konstante ungleich Null, $\mu$, verwendet, die einen gewünschten Wert der Differenz zwischen der stehenden und der ruhenden Herzfrequenz darstellt, um eine Wiederherstellungsbewertung für das Subjekt (S102) zu generieren, wobei $f(HR_{ruhend}, HR_{stehend})$ diese Form hat:

$$f(HR_{ruhend}, HR_{stehend}) = S \times \frac{A}{\sigma} \times B \left( \frac{(HR_{stehend} - HR_{ruhend}) - \mu}{\sigma} \right)^2,$$

wobei A und B Konstanten ungleich Null sind,
wobei $\mu$ eine Konstante ungleich Null ist, die einen gewünschten Wert der Differenz zwischen der stehenden und der ruhenden Herzfrequenz darstellt und als Ortsparameter fungiert, der die Spitze der Kurve der Verteilung, die durch die Wahrscheinlichkeitsdichtefunktion generiert wird, in einen Ort übersetzt, der eine optimale Wiederherstellungsbewertung für das Subjekt darstellt,
wobei S eine Skalierungskonstante ungleich Null ist und wobei $\sigma$ eine Skalierungskonstante ungleich Null ist, welche die Weite der Kurve der Verteilung definiert, die durch die Wahrscheinlichkeitsdichtefunktion generiert wird; und
Ausgeben der generierten Wiederherstellungsbewertung (S103).

**Revendications**

1. Procédé mis en œuvre par ordinateur permettant de calculer un score de récupération pour un sujet, le procédé comprenant :

l'obtention d'une paire de valeurs de fréquence cardiaque, une première des valeurs, $HR_{rest}$, représentant la fréquence cardiaque du sujet en position au repos et une seconde des valeurs, $HR_{stand}$, représentant la fréquence cardiaque du sujet en position debout (S101) ;

**caractérisé en ce que** le procédé comprend en outre :

l'entrée de la paire de valeurs de fréquence cardiaque dans une fonction de densité de probabilité $f(HR_{rest}, HR_{stand})$ qui utilise la différence entre la valeur de $(HR_{stand} - HR_{rest})$ et une constante non nulle, $\mu$, qui représente une valeur souhaitée de la différence entre les fréquences cardiaques en position debout et au repos, afin de générer un score de récupération pour le sujet (S102), dans lequel $f(HR_{rest}, HR_{stand})$ est de la forme suivante :

$$f(HR_{rest}, HR_{stand}) = S \times \frac{A}{\sigma} \times B \left( \frac{(HR_{stand} - HR_{rest}) - \mu}{\sigma} \right)^2,$$

dans laquelle A et B sont des constantes non nulles,
dans laquelle $\mu$ est une constante non nulle qui représente une valeur souhaitée de la différence entre les fréquences cardiaques en position debout et au repos, et agit comme un paramètre d'emplacement qui déplace par translation le pic de la courbe de la distribution générée par la fonction de densité de probabilité vers un emplacement représentant un score de récupération optimal pour le sujet,
dans laquelle S est une constante d'échelle non nulle, et
dans laquelle $\sigma$ est une constante d'échelle non nulle qui définit la largeur de la courbe de la distribution générée par la fonction de densité de probabilité ; et
la sortie du score de récupération généré (S103).

2. Procédé mis en œuvre par ordinateur selon la revendication **1,** dans lequel $\mu$ est proportionnelle à $\sigma$, facultativement dans lequel $\mu = 3\sigma$.

3. Procédé mis en œuvre par ordinateur selon l'une quelconque des revendications 1 à 2, dans lequel $\sigma$ est déterminée selon la différence maximale attendue entre les fréquences cardiaques en position debout et au repos pour le sujet, $OHR_{max}$, et la différence minimale attendue entre les fréquences cardiaques en position debout et au repos pour le sujet, $OHR_{min}$.

4. Procédé mis en œuvre par ordinateur selon la revendication 3, dans lequel $\sigma$ est déterminée en calculant $OHR_{max} - OHR_{min}$.

5. Procédé mis en œuvre par ordinateur selon la revendication 4, dans lequel $\sigma$ est déterminé en divisant $OHR_{max} - OHR_{min}$ par une constante non nulle C, facultativement dans lequel C représente le nombre d'écarts types nécessaires pour passer de $OHR_{min}$ à $\mu$ et de $\mu$ à $OHR_{max}$, facultativement dans lequel C = 6.

6. Procédé mis en œuvre par ordinateur selon l'une quelconque des revendications 3 à 5, dans lequel la différence entre $OHR_{max}$ et $OHR_{min}$ est entre 20 et 50.

7. Procédé mis en œuvre par ordinateur selon la revendication 6, dans lequel la différence entre $OHR_{max}$ et $OHR_{min}$ est entre 25 et 40, facultativement dans lequel la différence entre $OHR_{max}$ et $OHR_{min}$ est égale à 30, et facultativement dans lequel $OHR_{max} = 30$ et $OHR_{min} = 0$.

8. Procédé mis en œuvre par ordinateur selon une quelconque revendication précédente, dans lequel $B = e^{-\frac{1}{2}}$

9. Procédé mis en œuvre par ordinateur selon une quelconque revendication précédente, dans lequel $\mu = 3\sigma$, $\sigma = 5$, et facultativement dans lequel $S \times \frac{A}{\sigma} = 10$.

10. Procédé mis en œuvre par ordinateur selon l'une quelconque des revendications précédentes, dans lequel $S = P \times \frac{\sigma}{A}$ où P est une constante non nulle qui définit la limite supérieure du score de récupération, facultativement dans lequel $A = 1/\sqrt{2\pi}$, et dans lequel $S = P \times \sigma \times \sqrt{2\pi}$.

11. Procédé mis en œuvre par ordinateur selon une quelconque revendication précédente, dans lequel $\mu$ est entre 5 et 25, facultativement dans lequel $\mu$ est entre 10 et 20, et facultativement dans lequel $\mu$ est 15.

**12.** Procédé mis en œuvre par ordinateur selon une quelconque revendication précédente, dans lequel l'obtention de la paire de valeurs de fréquence cardiaque comprend :

l'obtention d'une première séquence d'échantillons de données de rythme cardiaque du sujet en position au repos et une seconde séquence d'échantillons de données de rythme cardiaque du sujet en position debout, dans lequel les échantillons de données de rythme cardiaque comprennent des valeurs d'intervalle interbattements, IBI, représentant le temps entre des battements de cœur successifs ;
le calcul de $HR_{rest}$ à partir de la première séquence d'échantillons de données de battements cardiaques ; et
le calcul de $HR_{stand}$ à partir de la seconde séquence d'échantillons de données de battements cardiaques.

**13.** Procédé mis en œuvre par ordinateur selon la revendication 12, dans lequel le calcul de $HR_{rest}$ consiste à diviser 60 000 par la valeur IBI moyenne en millisecondes pour la première séquence d'échantillons de données de rythme cardiaque, et dans lequel le calcul de $HR_{stand}$ comprend la division de 60 000 par la valeur IBI moyenne en millisecondes pour la seconde séquence d'échantillons de données de rythme cardiaque.

**14.** Support lisible par ordinateur sur lequel sont enregistrées des instructions qui, lorsqu'elles sont exécutées par un processeur, amènent le processeur à réaliser le procédé selon l'une quelconque revendication précédente.

**15.** Système de calcul d'un score de récupération pour un sujet, le système comprenant un processeur et une mémoire, la mémoire stockant des instructions qui, lorsqu'elles sont exécutées par le processeur, amènent le processeur à réaliser les opérations comprenant :

l'obtention d'une paire de valeurs de fréquence cardiaque, une première des valeurs, $HR_{rest}$, représentant la fréquence cardiaque de l'utilisateur en position au repos et une seconde des valeurs, $HR_{stand}$, représentant la fréquence cardiaque de l'utilisateur en position debout (S101) ;
**caractérisé en ce que** les opérations comprennent en outre :

l'entrée de la paire de valeurs de fréquence cardiaque dans une fonction de densité de probabilité $f(HR_{rest}, HR_{stand})$ qui utilise la différence entre $(HR_{stand} - HR_{rest})$ et une constante non nulle, $\mu$, qui représente une valeur souhaitée de la différence entre les fréquences cardiaques en position debout et au repos, afin de générer un score de récupération pour le sujet (S102), dans lequel $f(HR_{rest}, HR_{stand})$ est de la forme suivante :

$$f(HR_{rest}, HR_{stand}) = S \times \frac{A}{\sigma} \times B \left( \frac{(HR_{stand} - HR_{rest}) - \mu}{\sigma} \right)^2,$$

dans laquelle A et B sont des constantes non nulles,
dans laquelle $\mu$ est une constante non nulle qui représente une valeur souhaitée de la différence entre les fréquences cardiaques en position debout et au repos, et agit comme un paramètre d'emplacement qui déplace par translation le pic de la courbe de la distribution générée par la fonction de densité de probabilité vers un emplacement représentant un score de récupération optimal pour le sujet,
dans laquelle S est une constante d'échelle non nulle, et
dans laquelle $\sigma$ est une constante d'échelle non nulle qui définit la largeur de la courbe de la distribution générée par la fonction de densité de probabilité ; et
la sortie du score de récupération généré (S103).

**FIG. 1**

**FIG. 2**

**FIG. 3**

FIG. 4

EP 4 228 503 B1

**FIG.5**

171

139

169

175

173

177

**FIG. 6**

300 User Electronics Device

303
Camera

301
Display

305
Controller

306
User Input
Unit

308
TOF sensor

304
Memory

307
Communicator

309
IMU

**Fig. 7**

13:27

Recovery Reading
Part 1

✕

_ 302

_ 3021

Lie down, relax and
breath normally

3 MIN

_ 3022

START

Puck 5DOD

300

**Fig. 8**

13:19　　　　　　　　　　　　　　　.ıl 🗢 ■

Recovery Reading
Part 1　　　　　　　×

⌠ 302

3023

3025

02:58

300

**Fig. 9**

13:22

Recovery Reading
Part 2

✕

302

3027

Stand up, relax and
breath normally

3 MIN

300

**Fig. 10**

13:22

Recovery Reading
Part 2

✕

302

3029

300

3031

02:49

**Fig. 11**

13:25

Saturday, 03 October

RECOVERY SCORE

302

3033

8

3034

Train Normally

OHR

3035

♥ 116.1

HRV

3037

♥ 294.1

MENTAL STRESS

3039

Moderate

PHYSICAL STRAIN

3041

Safe

300

HEART HEALTH

3043

Improving

♥ Insights

Account

**Fig. 12**

3045

**Fig. 13**

**Fig. 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160066840 A1
- US 20150182129 A1

- US 20160143579 A1

**Non-patent literature cited in the description**

- **PAN, JIAPU** ; **TOMPKINS, WILLIS J**. A Real-Time QRS Detection Algorithm. *IEEE Transactions on Biomedical Engineering. BME*, March 1985, vol. 32 (3), 230-236